# EUROPEAN PATENT SPECIFICATION

(11) **EP 3 944 854 B1**
(45) Date of publication and mention of the grant of the patent: **23.09.2026**
(21) Application number: 21187933.3
(22) Date of filing: 27.07.2021
(51) Int. Cl.: A61K 8/34, A61K 8/92, A61Q 13/00, A61K 8/64, A61K 8/49, A61K 8/46, A61K 8/37, A61K 8/35, C11B 9/00, G01N 33/50, G01N 33/74, A61L 9/01

(54) **USE OF A FRAGRANCE COMPOSITION FOR SUPPRESSING AN UNPLEASANT ODOR.**
VERWENDUNG EINER DUFTKOMPOSITION ZUR UNTERDRÜCKUNG EINES UNANGENEHMEN GERUCHS
UTILISATION D'UNE COMPOSITION PARFUMÉE POUR SUPPRIMER UNE ODEUR DÉSAGRÉABLE

(30) Priority: 28.07.2020 JP 2020127591; 16.10.2020 JP 2020174788
(43) Date of publication of application: 02.02.2022
(73) Proprietor: Takasago International Corporation, Tokyo 144-8721 (JP)
(72) Inventor: Mihara, Hisashi, Kanagawa, 254-0073 (JP); Kuwahara, Yukari, Kanagawa, 254-0073 (JP); Murai, Masato, Kanagawa, 254-0073 (JP); Kobayashi, Tsuyoshi, Kanagawa, 254-0073 (JP); Ishida, Kenya, Kanagawa, 254-0073 (JP)
(74) Representative: Hoffmann Eitle

(56) References cited:
- EP-A1- 1 793 796
- JP-A- 2000 281 509
- JP-A- 2003 190 264
- RU-C2- 2 592 351
- US-A1- 2013 303 432
- US-A1- 2017 181 935
- US-A1- 2017 196 786
- US-A1- 2020 056 118
- US-B2- 8 741 275
- VERBEURGT CHRISTOPHE ET AL: "Profiling of Olfactory Receptor Gene Expression in Whole Human Olfactory Mucosa", PLOS ONE, vol. 9, no. 5, 1 May 2014 (2014-05-01), pages e96333, XP055877455, DOI: 10.1371/journal.pone.0096333
- REDDY GAUTAM ET AL: "Antagonism in olfactory receptor neurons and its implications for the perception of odor mixtures", vol. 7, 1 January 2018 (2018-01-01), XP055878098, Retrieved from the Internet <URL:https://elifesciences.org/articles/34958.pdf> [retrieved on 20220111], DOI: 10.7554/eLife.34958
- MARKT SARAH C ET AL: "Sniffing out significant "Pee values": genome wide association study of asparagus anosmia", 1 January 2016 (2016-01-01), pages i6071, XP055878095, Retrieved from the Internet <URL:https://www.bmj.com/content/355/bmj.i6071.full.pdf> DOI: 10.1136/bmj.i6071
- COOKE M. ET AL: "Time profile of putrescine, cadaverine, indole and skatole in human saliva", ARCHIVES OF ORAL BIOLOGY, vol. 48, no. 4, 1 April 2003 (2003-04-01), GB, pages 323 - 327, XP055878125, ISSN: 0003-9969, DOI: 10.1016/S0003-9969(03)00015-3

## Description

### TECHNICAL FIELD

The present invention relates to a use of a fragrance composition containing a fragrance component for suppressing an unpleasant odor.

### BACKGROUND ART

Recently, the population of persons sensitive to bad smells and unpleasant odors around them is growing depending on diversification of consumer preferences and living environments.

Typical examples of substances that cause unpleasant odors in living spaces include skatole and indole which are contained in body odor, fecal odor, rotten odor, etc. (Patent Literature 1).

Regarding techniques of selectively masking these unpleasant odors, the possibility of masking methods based on the antagonism mechanism, wherein an olfactory receptor responsive to a specific odorous substance is searched and activation thereof is suppressed by a specific substance, is becoming clear. As olfactory receptors that respond to skatole or indole that is an unpleasant odor-causing substance, OR2W1, OR5P3, OR5K1 and OR8H1 are disclosed, and it is described that skatole odor or indole odor can be suppressed by suppressing responses of these olfactory receptors (Patent Literature 2).

It is said that, in this conventional means, recognition of an unpleasant odor is specifically suppressed by inhibiting an olfactory receptor that responds to skatole or indole with a specific odor molecule. However, masking effects thereof are insufficient in terms of sense. Further, since there are many olfactory receptors in mammals, it has been continuously desired to specify olfactory receptors relative to odor molecules.

### CITATION LIST

### PATENT LITERATURES

Patent Literature 1: Japanese Laid-Open Patent Publication No. 2008-036434
Patent Literature 2: Japanese Laid-Open Patent Publication No. 2012-250958

### NON-PATENT DOCUMENTS

Non-Patent Literature 1: Nature Communications 10:209 (2019)

### SUMMARY OF INVENTION

### TECHNICAL PROBLEM

Many of olfactory receptors whose responses to skatole and indole are disclosed are broadly tuned olfactory receptors, and it has been unclear and unknown which of olfactory receptors characterizes the recognition of unpleasant odor. Further, olfactory receptors which clearly respond to α-terpineol, 4-terpineol and p-methylacetophenone have been unknown. Under such circumstances, it is desired to provide: a method for efficiently screening a candidate substance for an unpleasant odor masking agent by searching an olfactory receptor which responds to an unpleasant odor-causing substance typified by skatole, indole, α-terpineol, 4-terpineol and p-methylacetophenone; and a fragrance composition capable of masking an unpleasant odor for reducing unpleasantness of the unpleasant odor.

The present disclosure further relates to a method for screening an unpleasant odor masking agent for an unpleasant odor selected from the group consisting of skatole odor, indole odor, α-terpineol odor, 4-terpineol odor and p-methylacetophenone odor, and an unpleasant odor masking composition for reducing unpleasantness of at least one of fecal odor, body odor, tobacco odor, breath odor and musty odor.

### SOLUTION TO PROBLEM

The present inventor diligently made researches in order to solve the above-described problems and succeeded in newly identifying olfactory receptors which respectively respond to skatole, indole, α-terpineol, 4-terpineol and p-methylacetophenone. The present inventor further made researches and found that evaluation and selection of an unpleasant odor masking agent for fecal odor, body odor, tobacco odor, breath odor or musty odor with use of masking effects of an olfactory receptor antagonist can be carried out by using these olfactory receptors, and also found that antagonist candidate compounds that suppress the response of OR2L3 have excellent effects of suppressing unpleasantness of at least one of fecal odor, body odor, tobacco odor, breath odor and musty odor. Moreover, surprisingly, it was found that superior effects of suppressing at least one of fecal odor, body odor, tobacco odor, breath odor and musty odor are exerted by combined use of a specific component among fragrances which do not exert the effect of suppressing the response of OR2L3.

Specifically, the present invention provides a use of a fragrance composition for suppressing an unpleasant odor described below.
[1] A use of a fragrance composition for suppressing a response strength of at least one olfactory receptor polypeptide, which is selected from the group consisting of OR2L3 and polypeptides that comprise an amino acid sequence having at least 80% identity to an amino acid sequence of OR2L3 and are responsive to an unpleasant odor-causing substance, to the unpleasant odor-causing substance, wherein the fragrance composition comprises a fragrance component comprising at least one selected from the group consisting of materials of Group A below:
   Group A: methyl fenchol, ethyl 3-mercapto-2-methylbutyrate, dimethyl-2-(1-phenylethyl)cyclopropylmethanol, 5-cyclohexadecenone, 1-(2,2,6-trimethylcyclohexyl)hexan-3-ol, 2-methyl-4-(2,2,3-trimethyl-3-cyclopentenyl)-2-buten-1-ol, p-mentha-8-thiol-3-one, cardamom oil, clary sage oil, mandarin oil and spearmint oil.
[2] The use according to item [1], wherein the unpleasant odor-causing substance is at least one selected from the group consisting of skatole, indole, α-terpineol, 4-terpineol and p-methylacetophenone.
[3] The use according to item [1] or [2], wherein the unpleasant odor is at least one selected from the group consisting of fecal odor, body odor, tobacco odor, breath odor and musty odor.
[4] The use according to any one of items [1] to [3], wherein the fragrance component further contains at least one selected from the group consisting of materials of Group B below:
   Group B: methyl-2-nonynoate, p-mentha-1,8-diene-7-al, 2-phenylpropanal, ethyl amyl ketone, cis-2-methyl-4-propyl-1,3-oxathiane, ethyl hexanoate, ethyl 2-methylvalerate, tetrahydro-4-methyl-2-(2-methylpropenyl)-2H-pyran, (2S,4R)-tetrahydro-4-methyl-2-(2-methylpropenyl)-2H-pyran, allyl hexanoate, isoamyl acetate, 3-methyl-2-(cis-2-pentenyl)-2-cyclopenten-1-one, cis-3-hexenyl acetate, prenyl acetate, 2-methoxynaphthalene, cis-3-hexen-1-ol, ethyl isovalerate, grapefruit oil, chamomile oil, lime oil, galbanum oil, citronella oil, peppermint oil, eucalyptus oil, nutmeg oil and coriander oil.
[5] The use according to item [4], wherein the fragrance composition comprises 0.000001 to 50% by mass of the fragrance component of Group A and 0.0001 to 50% by mass of the fragrance component of Group B.

### ADVANTAGEOUS EFFECTS OF INVENTION

By using the method of the present disclosure, a candidate substance for an unpleasant odor masking agent for skatole odor, indole odor, α-terpineol odor, 4-terpineol odor or p-methylacetophenone odor can be screened. It is expected that the screening method of the present disclosure can contribute to the development of an unpleasant odor masking agent for skatole odor, indole odor, α-terpineol odor, 4-terpineol odor or p-methylacetophenone odor.

Further, when many odors of candidate substances are evaluated only based on human olfaction for developing a new fragrance material, there are problems regarding olfactory fatigue, differences among individuals, etc., and it may be difficult to appropriately select a candidate substance. According to the method of the present disclosure, these problems can be solved or reduced.

Moreover, by using antagonist candidate compounds that suppress the response of OR2L3 or a polypeptide which comprise an amino acid sequence having at least 80% identity to an amino acid sequence of OR2L3 and is responsive to the unpleasant odor-causing substance, in combination with specific compounds conventionally used as fragrance, etc., it is possible to provide superior effects of suppressing an unpleasant odor when compared to the case of using only the antagonist candidate compounds.

### BRIEF DESCRIPTION OF DRAWINGS

FIG. 1 shows measurement results of the response of the olfactory receptor OR2L3 to skatole.
FIG. 2 shows measurement results of the response of the olfactory receptor OR2L3 to indole.
FIG. 3 shows measurement results of the response of the olfactory receptor OR2L3 to α-terpineol.
FIG. 4 shows measurement results of the response of the olfactory receptor OR2L3 to 4-terpineol.
FIG. 5 shows measurement results of the response of the olfactory receptor OR2L3 to p-methylacetophenone.
FIG. 6 shows the effect of suppressing the response of the olfactory receptor OR2L3 to skatole obtained by the addition of Guava coeur (registered trademark).
FIG. 7 shows the effect of suppressing the response of the olfactory receptor OR2L3 to skatole obtained by the addition of Dextramber (registered trademark).
FIG. 8 shows the effect of suppressing the response of the olfactory receptor OR2L3 to skatole obtained by the addition of Hindinol (registered trademark).
FIG. 9 shows the effect of suppressing the response of the olfactory receptor OR2L3 to indole obtained by the addition of Guava coeur (registered trademark).
FIG. 10 shows the effect of suppressing the response of the olfactory receptor OR2L3 to indole obtained by the addition of Dextramber (registered trademark).
FIG. 11 shows the effect of suppressing the response of the olfactory receptor OR2L3 to indole obtained by the addition of Hindinol (registered trademark).

### DESCRIPTION OF EMBODIMENTS

Hereinafter, the screening method used in the present disclosure will be described, and then the use of the fragrance composition for suppressing an unpleasant odor of the present invention will be specifically described.

### 1. Screening method

The screening method used in the present disclosure is characterized in that it is a method for screening a candidate substance for an unpleasant odor masking agent from among test substances using an olfactory receptor OR2L3 that is responsive to an unpleasant odor-causing substance, which includes the steps of:
(i) bringing the unpleasant odor-causing substance into contact with the olfactory receptor (olfactory receptor polypeptide) selected from the group consisting of OR2L3 and proteins (polypeptides) which comprise an amino acid sequence having at least 80% identity to an amino acid sequence of OR2L3 and are responsive to the unpleasant odor-causing substance to measure a response of the olfactory receptor to the unpleasant odor-causing substance;
(ii) measuring a response of the olfactory receptor used in the step (i) in the absence of the unpleasant odor-causing substance;
(iii) obtaining a change value of responsiveness by making a calculation based on comparison between measurement results in the steps (i) and (ii);
(iv) mixing each of the test substances separately with the unpleasant odor-causing substance in the step (i) and obtaining a change value of responsiveness by making a calculation in a manner similar to that in the step (iii); and
(v) selecting a test substance, regarding which the value of the step (iv) is reduced when compared to the value of the step (iii), as the candidate substance for the unpleasant odor masking agent.

In the above-described screening method, a candidate substance for an unpleasant odor masking agent is selected from among test substances, using the responsiveness of the test substances to an olfactory receptor selected from the group consisting of an olfactory receptor OR2L3 and proteins (polypeptides) which comprise an amino acid sequence having at least 80% identity to an amino acid sequence of OR2L3 and are responsive to an unpleasant odor-causing substance as an index.

It is known that when one odorous substance activates a plurality of olfactory receptors, emotions and behaviors such as likes or dislikes and attraction or avoidance are generally caused. Non-Patent Literature 1 shows that the "quality" of an odor is defined in each olfactory receptor. When the response of OR2L3 to several hundred types of odorous substances was measured and diligently researched and summarized, since substances responsive to OR2L3 include a certain number of unpleasant odor substances, OR2L3 was associated with recognition of unpleasant odors corresponding to dislikes and avoidance. Accordingly, by evaluating the responsiveness of test substances to the olfactory receptor OR2L3, a candidate substance which can inhibit bonding between an unpleasant odor-causing substance and the olfactory receptor can be selected from among the test substances.

Note that in this specification, the "test substance" is not particularly limited, but means a target to be researched with respect to the unpleasant odor masking effect and means a compound, composition or mixture. Further, in this specification, the "unpleasant odor masking agent" is not particularly limited, but means a compound, composition or mixture capable of masking an unpleasant odor. Hereinafter, each step of the screening method used in the present invention will be described.

### <Step (i)>

In the step (i), an unpleasant odor-causing substance is brought into contact with an olfactory receptor selected from the group consisting of OR2L3 and proteins (polypeptides) which comprise an amino acid sequence having at least 80% identity to an amino acid sequence of OR2L3 and are responsive to the unpleasant odor-causing substance to measure a response of the olfactory receptor to the unpleasant odor-causing substance.

As the olfactory receptor, one selected from the group consisting of OR2L3 and proteins (polypeptides) which comprise an amino acid sequence having at least 80% identity to an amino acid sequence of OR2L3 and are responsive to the unpleasant odor-causing substance is used.

OR2L3 has been registered in GenBank as NM_001004687 and is a protein (polypeptide) consisting of an amino acid sequence (SEQ ID NO: 2) encoded by DNA of a nucleotide sequence represented by SEQ ID NO: 1.

Since the olfactory receptor OR2L3 selectively responds to a certain type of unpleasant odor substance typified by skatole, it is expected that the screening method using OR2L3 can contribute to the development of unpleasant odor masking agents.

As the olfactory receptor, one selected from the group consisting of proteins (polypeptides) which comprise an amino acid sequence having at least 80%, preferably at least 85%, more preferably at least 90%, even more preferably at least 95%, and particularly preferably at least 98% identity to an amino acid sequence of OR2L3 and are responsive to an unpleasant odor-causing substance may be used. Note that in this specification, the sequence identity of an amino acid sequence is calculated using the BLAST search algorithm (publicly available from NCBI).

As the olfactory receptor, one material may be used solely, or two or more materials may be used in combination.

In the present disclosure, the unpleasant odor-causing substance is fecal odor, body odor, tobacco odor, breath odor and musty odor typified by skatole, indole, α-terpineol, 4-terpineol or p-methylacetophenone, and means an OR2L3-responsive compound, composition or mixture that is unpleasant for humans.

In the present disclosure, the method of bringing the unpleasant odor-causing substance into contact with the olfactory receptor to measure the response of the olfactory receptor to the unpleasant odor-causing substance is not particularly limited. For example, the response of the olfactory receptor may be measured by carrying out the contact with the unpleasant odor-causing substance on a cell isolated from a living body in which the olfactory receptor is expressed. Alternatively, the response of the olfactory receptor may be measured by carrying out the contact with the unpleasant odor-causing substance on a cell in which the olfactory receptor is artificially expressed by means of genetic engineering. The time for the contact between the olfactory receptor and the unpleasant odor-causing substance cannot be categorically described because it depends on the concentration of the unpleasant odor-causing substance and the measurement method, but the response may be measured immediately after the contact. Generally, in the reporter gene assay method, the time is 0 to 4 hours, and preferably 2 to 4 hours, and in the calcium imaging method, the time is about several seconds to several minutes.

The cell in which the olfactory receptor is artificially expressed by means of genetic engineering can be prepared by transforming a cell with use of a vector in which a gene encoding the olfactory receptor is incorporated.

In a preferred embodiment of the present invention, the N-terminal 20 amino acid residues of bovine rhodopsin may be incorporated together with the olfactory receptor. By incorporation of the N-terminal 20 amino acid residues of bovine rhodopsin, cell membrane expression of the olfactory receptor can be promoted.

Bovine rhodopsin has been registered in GenBank as NM_001014890. Bovine rhodopsin is a protein (polypeptide) consisting of an amino acid sequence (SEQ ID NO: 4) encoded by DNA of from position 1 to position 1047 of a nucleotide sequence represented by SEQ ID NO: 3.

Further, instead of bovine rhodopsin, a protein (polypeptide) which comprises an amino acid sequence having at least 80%, preferably at least 85%, more preferably at least 90%, even more preferably at least 95%, and particularly preferably at least 98% identity to the amino acid sequence represented by SEQ ID NO: 4 and can promote cell membrane expression of the olfactory receptor may be used.

Note that amino acid residues of a protein (polypeptide) other than bovine rhodopsin may also be used as long as it can promote cell membrane expression of the olfactory receptor.

The method for measuring the response of the olfactory receptor is not particularly limited, and any method used in the art may be used. For example, it is known that when an aroma compound binds to an olfactory receptor, a G-protein in a cell is activated, the G-protein activates adenylate cyclase to convert ATP into cyclic AMP (cAMP), and the amount of cAMP in the cell is increased thereby. Accordingly, by measuring the amount of cAMP, the response of the olfactory receptor can be measured. As the method for measuring the amount of cAMP, an ELISA method, a reporter gene assay method or the like is used. It is particularly preferred that the response of the olfactory receptor is measured by a reporter gene assay method in which a luminescent substance such as luciferase is used.

### <Step (ii)>

In the step (ii), the response of the olfactory receptor used in the step (i) is measured in the absence of the unpleasant odor-causing substance.

As the method for measuring the response of the olfactory receptor, the same method as that in the step (i) can be used, except that the unpleasant odor-causing substance is not brought into contact with the olfactory receptor. For example, the response of the olfactory receptor may be measured on a cell isolated from a living body in which the olfactory receptor is expressed. Alternatively, the response of the olfactory receptor may be measured on a cell in which the olfactory receptor is artificially expressed by means of genetic engineering. For appropriate comparison between measurement results in the steps (i) and (ii), measurement conditions in the steps (i) and (ii) are preferably the same except for the presence or absence of contact with the unpleasant odor-causing substance.

### <Step (iii)>

**In** the step (iii), a change value of responsiveness is obtained by making a calculation based on comparison between measurement results in the steps (i) and (ii).

According to one embodiment of the present invention, the change of responsiveness may be evaluated based on a Fold Increase value, which is obtained by dividing the measurement result in the step (i) by the measurement result in the step (ii), as an index. For example, when the response of the olfactory receptor is measured by a reporter gene assay method in which a luminescent substance such as luciferase is used, the evaluation can be made using an unpleasant odor-causing substance having a concentration with which the Fold Increase value becomes preferably at least 2, more preferably at least 4, and even more preferably at least 10.

### <Step (iv)>

In the step (iv), test substances are mixed with the unpleasant odor-causing substance in the step (i) and a change value of responsiveness is obtained by making a calculation in a manner similar to that in the step (iii).

### <Step (v)>

In the step (v), a test substance, regarding which the value of the step (iv) is reduced when compared to the value of the step (iii), is selected as a candidate substance for the unpleasant odor masking agent.

In the present invention, when the measurement results in the steps (iii) and (iv) are compared to each other and reduction in the change value of responsiveness is shown, the test substance used in the step (iv) can be evaluated as a candidate substance for the unpleasant odor masking agent.

In the above-described manner, a candidate substance for the unpleasant odor masking agent can be screened from test substances. According to the above-described screening method, a candidate substance for the unpleasant odor masking agent can be selected from many test substances without problems caused by organoleptic evaluation, such as olfactory fatigue and individual difference.

The selected substance can be used as the candidate substance for the unpleasant odor masking agent. Based on the selected substance, modification or the like can be carried out according to need to develop a novel compound having an optimum odor. Moreover, by blending the selected substance with another fragrance material, an unpleasant odor can be suppressed and a fragrance material having an optimum odor can be developed. Use of the above-described screening method can contribute to the development of a novel fragrance material for the unpleasant odor masking agent.

### 2. Fragrance composition

By using the above-described screening method, an unpleasant odor masking agent appropriate for an unpleasant odor-causing substance can be efficiently selected, and by using the unpleasant odor masking agent as an active component, a fragrance composition (also referred to as "unpleasant odor masking composition") having an intended effect of suppressing an unpleasant odor can be obtained.

The fragrance composition used in the present invention contains, as an active component, at least one selected from Group (A) described below.

Further, in a preferred embodiment of the present invention, the fragrance composition used in the present invention contains, as active components, at least one selected from Group (A) described below and at least one selected from Group (B) described below.

In this regard, Group (A) is a group of antagonist candidate compounds that suppress the response of OR2L3 found out as unpleasant odor masking agents according to the above-described screening method. Group (B) is a group of compounds which do not exert the effect of suppressing the response of OR2L3 in the above-described screening method but may exert the effect of enhancing the effect of suppressing an unpleasant odor had by Group (A) by use in combination with Group (A).

Examples of Group (A) include methyl fenchol (Humus Ether (registered trademark)), ethyl 3-mercapto-2-methylbutyrate (Guava coeur (registered trademark)), dimethyl-2-(1-phenylethyl)cyclopropylmethanol (Pamplefix (registered trademark)), 5-cyclohexadecenone (Ambretone (registered trademark)), 1-(2,2,6-trimethylcyclohexyl)hexan-3-ol (Dextramber (registered trademark)), 2-methyl-4-(2,2,3-trimethyl-3-cyclopentenyl)-2-buten-1-ol (Hindinol (registered trademark)), p-mentha-8-thiol-3-one (Ringonol (registered trademark)), cardamom oil, clary sage oil, mandarin oil and spearmint oil.

The blending amount of Group (A) in the fragrance composition of the present invention is not limited by the type of a product or scent, but it is preferably about 0.000001 to 50% by mass, and for example, it is preferably 0.0001 to 50% by mass. The preferred blending amount of Group (A) varies depending on the threshold.

For example, in the case of a compound having a low threshold such as Guava coeur (registered trademark) and Ringonol (registered trademark), the blending amount is preferably 0.000001% by mass or more, more preferably 0.00001 to 0.1% by mass, and even more preferably 0.00001 to 0.001% by mass. For example, it is preferably 0.00001% by mass or more, more preferably 0.0001 to 1% by mass, and even more preferably 0.0001 to 0.01% by mass.

In the case of other materials such as Ambretone (registered trademark), Dextramber (registered trademark) and clary sage oil, the blending amount is preferably 0.01% by mass or more, more preferably 0.1 to 50% by mass, and even more preferably 0.1 to 20% by mass. For example, it is preferably 0.1% by mass or more, more preferably 0.1 to 50% by mass, and even more preferably 0.1 to 20% by mass.

Examples of Group (B) include methyl-2-nonynoate, p-mentha-1,8-diene-7-al, 2-phenylpropanal, ethyl amyl ketone, cis-2-methyl-4-propyl-1,3-oxathiane, ethyl hexanoate, ethyl 2-methylvalerate, tetrahydro-4-methyl-2-(2-methylpropenyl)-2H-pyran, (2S,4R)-tetrahydro-4-methyl-2-(2-methylpropenyl)-2H-pyran, allyl hexanoate, isoamyl acetate, 3-methyl-2-(cis-2-pentenyl)-2-cyclopenten-1-one, cis-3-hexenyl acetate, prenyl acetate, 2-methoxynaphthalene, cis-3-hexen-1-ol, ethyl isovalerate, grapefruit oil, chamomile oil, lime oil, galbanum oil, citronella oil, peppermint oil, eucalyptus oil, nutmeg oil and coriander oil.

The blending amount of Group (B) in the fragrance composition of the present invention is not limited by the type of a product or scent, but it is preferably about 0.0001 to 50% by mass. For example, it is preferably 0.001 to 50% by mass, more preferably 0.001 to 30% by mass, and even more preferably 0.01 to 20% by mass.

When Group A and Group B are used in combination, in the case where Group A is a compound having a low threshold such as Guava coeur (registered trademark), Group A: Group B (mass reference) is preferably 0.0005%:99.9995% to 50%:50%, more preferably 1%:99% to 40%:60%, and even more preferably 10%:90% to 30%:70%. In the case where Group A is clary sage oil or the like, Group A:Group B (mass reference) is preferably 1%:99% to 99%:1%, more preferably 10%:90% to 90%:10%, and even more preferably 30%:70% to 70%:30%.

The fragrance composition of the present invention is suitably used as a fragrance composition for suppressing an unpleasant odor.

According to a preferred embodiment of the present invention, the above-described fragrance composition containing Group A and Group B is used for suppressing unpleasantness of at least one of fecal odor, body odor, tobacco odor, breath odor and musty odor, each of which is an unpleasant odor composed of at least one selected from the group consisting of skatole, indole, α-terpineol, 4-terpineol and p-methylacetophenone.

A publicly-known fragrance or a commonly-used additive explained below can be blended in the fragrance composition used in the present invention within a range in which the effects of the present invention are not reduced, i.e., within quantitative and qualitative ranges with which unpleasantness associated with at least one unpleasant odor selected from the group consisting of fecal odor, body odor, tobacco odor, breath odor and musty odor can be relieved.

Examples of the publicly-known fragrance include: hydrocarbons such as α-pinene, limonene and cedrene; aliphatic saturated or unsaturated alcohols such as 1-octen-3-ol and nonanol; saturated or unsaturated chain terpene alcohols such as myrcenol, tetrahydro linalool and nerol; cyclic terpene alcohols such as isopulegol and bornyl methoxy cyclohexanol; sesquiterpene alcohols such as farnesol; aromatic alcohols such as γ-phenylpropyl alcohol and dimethylbenzylcarbinol; aliphatic ketones such as aliphatic aldehyde, terpene aldehyde, aromatic aldehyde and Iso E Super (7-acetyl-1,2,3,4,5,6,7,8-octahydro-1,1,6,7-tetramethylnaphthalene); terpene-based cyclic ketones; cyclic ketones; aliphatic esters; acetic acid esters; propionic acid esters; butyric acid esters; benzoic acid esters; phenylacetic acid esters; salicylic acid esters; and anthranilic acid esters. The blending amount of the publicly-known fragrance in the fragrance composition can be suitably selected depending on the type, purpose or the like of the fragrance to be blended and is not particularly limited.

The fragrance composition used in the present invention can be blended in perfumery and cosmetics, etc. together with the commonly-used additive depending on the form or dosage form thereof.

Examples of the commonly-used additive include publicly-known ultraviolet absorbers; fresheners such as menthol and methyl salicylate; and skin stimulants such as capsicum tincture and nonyl vanillylamide. The blending amount of the commonly-used additive is not particularly limited, but it is about 0.005 to 5.0% by mass relative to the whole fragrance composition used in the present invention.

### 3. Product containing fragrance composition

Examples of the product containing the fragrance composition used in the present invention include fragrance products, perfumery and cosmetics, basic skin care products, makeup cosmetics, hair cosmetics, cosmetics for suntan, medicinal cosmetics, hair care products, soaps, body cleaning agents, bath agents, laundry detergents, fabric softeners, cleaning agents, kitchen detergents, bleaching agents, aerosols, air fresheners, repellents and general goods.

For example, examples of fragrance products include perfumes, eau de parfum, eau de toilette and eau de cologne; examples of basic skin care products include face wash creams, vanishing creams, cleansing creams, cold creams, massage creams, emulsions, lotions, beauty liquids, packs and makeup removers;
examples of makeup cosmetics include foundations, lipsticks and lip balms; examples of hair cosmetics include hair tonics, hair styling liquids and hair sprays;
examples of cosmetics for suntan include suntan products and sunscreen products; and
examples of medicinal cosmetics include antiperspirants, after-shave lotions and gels, permanent wave agents, medicated soaps, medicated shampoos and medicated skin cosmetics.

Examples of hair care products include shampoos, rinses, two-in-one shampoos, conditioners, treatments and hair packs;
examples of soaps include toilet soaps and bath soaps;
examples of body cleaning agents include body soaps, body shampoos and hand soaps; and
examples of bath agents include bath additives (bath salts, bath tablets, bath liquids, etc.), foam baths (bubble baths, etc.), bath oils (bath perfumes, bath capsules, etc.), milk baths, bath jellies and bath cubes.

Examples of laundry detergents include heavy duty detergents for clothes, light duty detergents for clothes, liquid detergents, laundry soaps, compact detergents and powder soaps;
examples of fabric softeners include softeners and furniture cares;
examples of cleaning agents include cleansers, house cleaning agents, toilet cleaning agents, bath room cleaning agents, glass cleaners, mold removers and drainpipe cleaning agents;
examples of kitchen detergents include kitchen soaps, kitchen synthetic soaps and dish detergents;
examples of bleaching agents include oxidation-type bleaching agents (chlorine-based bleaching agents, oxygen-based bleaching agents, etc.), reduction-type bleaching agents (sulfur-based bleaching agents, etc.) and optical bleaching agents;
examples of aerosols include spray-type aerosols and powder sprays;
examples of air fresheners include solid-type air fresheners, gel-type air fresheners and liquid-type air fresheners; and
examples of general goods include those in various forms such as tissue paper and toilet paper.

The content of the fragrance composition in the product can be suitably selected depending on the form or purpose of the product and is not particularly limited, but based on the mass of the product, it is preferably 0.01 to 100% by mass, more preferably 0.05 to 50% by mass, and even more preferably 0.1 to 20% by mass. For example, the content of the fragrance composition may be 0.01 to 30% by mass based on the mass of the product.

When using the fragrance composition of the present invention for the aforementioned product, depending on its purpose, any form can be selected, and examples of such forms include: an unprocessed state; a liquid state in which it is dissolved in an alcohol or a polyhydric alcohol such as propylene glycol and glycerin; a solubilization state or dispersion state in which it is solubilized or dispersed using a surfactant such as a nonionic surfactant, an anionic surfactant, a cationic surfactant and an amphoteric surfactant; and a microcapsule state obtained by treating it with an encapsulating agent.

Moreover, in some cases, the fragrance composition may be used in a stabilized and sustained-release form by forming an inclusion complex using an inclusion agent such as cyclodextrin. These are suitably selected to be appropriate for the form of a finished product such as a liquid form, a solid form, a powder form, a gel form, a mist form and an aerosol form.

Furthermore, it is used as a pro-fragrance, which itself is not an odorant but has the ability to release the fragrance composition under use/applicable conditions. **In** this case, the blending amount of the fragrance composition is not particularly limited, but it is about 0.001 to 20.0% by mass relative to the whole product.

### 4. Method for suppressing unpleasant odor

By using the fragrance composition of the present invention, at least one unpleasant odor selected from the group consisting of fecal odor, body odor, tobacco odor, breath odor and musty odor can be suppressed.

Specifically, the present invention provides a method for suppressing at least one unpleasant odor selected from the group consisting of fecal odor, body odor, tobacco odor, breath odor and musty odor, the method including suppressing said at least one unpleasant odor selected from the group consisting of fecal odor, body odor, tobacco odor, breath odor and musty odor by applying the fragrance composition of the present invention thereto.

The method for applying the fragrance composition of the present invention to the unpleasant odor is not particularly limited. For example, by using the aforementioned product containing the fragrance composition, the fragrance composition of the present invention can be applied to the unpleasant odor. The method for using it can be suitably selected depending on the form of the product.

### EXAMPLES

Hereinafter, the present invention will be described in more detail by way of examples. However, the present invention is not limited to the examples. Unless otherwise specified, the descriptions related to the composition ratio and the dilution concentration are based on the mass.

### [Example 1]

### Confirmation of response of olfactory receptor OR2L3 to unpleasant odor-causing substance

### (1) Cloning of olfactory receptor gene

A human olfactory receptor gene was obtained by cloning according to the PCR method using Human Genomic DNA: Female (Promega) based on the sequence information registered in GenBank. The N-terminal 20 amino acid residues of bovine rhodopsin were incorporated into a pME18S vector, and the obtained human olfactory receptor gene was further incorporated downstream thereof, thereby obtaining a human olfactory receptor gene expression vector.

### (2) Expression of olfactory receptor gene in HEK293T cells

0.05 µg of the human olfactory receptor gene expression vector, 0.01 µg of an RTP1S vector, 0.01 µg of a firefly luciferase vector pGL4.29 containing a cAMP-responsive sequence promoter (Promega), and 0.005 µg of a Renilla luciferase vector pGL4.74 containing a thymidine kinase promoter (Promega) were dissolved in 10 µL of Opti-MEM I (gibco) to obtain a gene solution (for one well). 100 µL of HEK293T cells were seeded in each well of a 96-well plate (Biocoat manufactured by Corning) so as to reach confluency after 24 hours. According to the directions for use of Lipofectamine 3000, by the lipofection method, the gene solution was added to each well to perform gene transfer to cells, and culturing was performed under 5% carbon dioxide atmosphere at 37°C for 24 hours.

### (3) Luciferase reporter gene assay

After removing the culture medium, 50 µL of CD293 medium (gibco) (20 µM L-glutamine added) including measured concentration of an aroma compound as a sample was added to each well, and stimulation was performed for 3 hours. After that, the luciferase activity was measured according to the directions for use of Dual-Luciferase Reporter Assay System (Promega). The response strength of the olfactory receptor was expressed by the Fold Increase value, which is obtained by dividing the luciferase activity generated by stimulation of the aroma compound by the luciferase activity generated in the test system not including the aroma compound.

### (4) Identification of olfactory receptor responsive to unpleasant odor substance

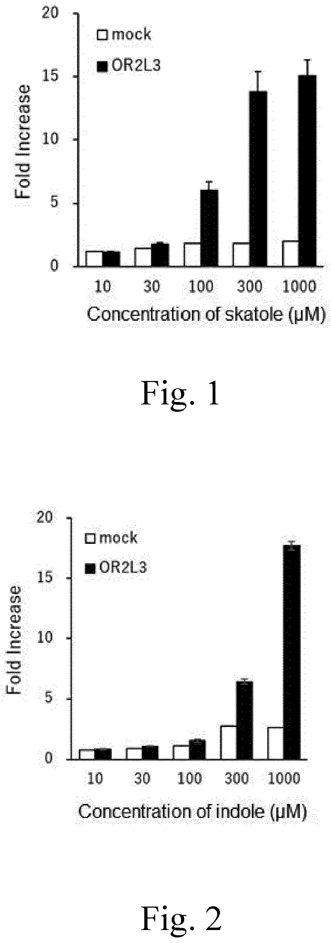

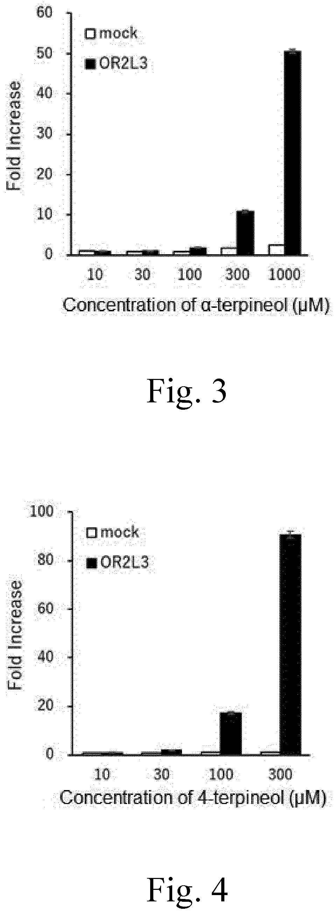

Responses of the olfactory receptor OR2L3 to skatole, indole, α-terpineol, 4-terpineol or p-methylacetophenone as a typical example of the unpleasant odor-causing substance were measured with various concentrations by means of luciferase reporter gene assay. The results are shown in Figures 1 to 5. OR2L3 showed concentration-dependent responses to the respective unpleasant odor-causing substances. Meanwhile, in the Mock test (using cells in which OR2L3 was not expressed), no response was shown. That is, it was shown that OR2L3 specifically responds to the respective unpleasant odor-causing substances.

### [Example 2]

### <Evaluation of effect of suppressing response of OR2L3 with respect to unpleasant odor masking agent>

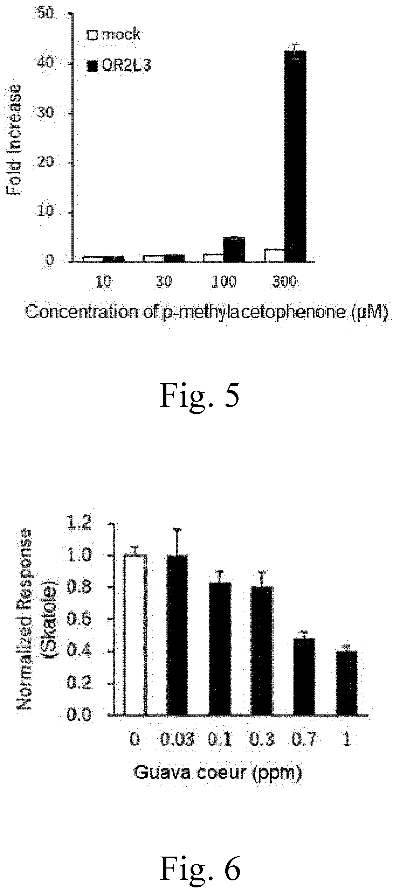

With respect to Group A, the effect of suppressing the response of OR2L3 that strongly responded to unpleasant odors was measured by means of luciferase reporter gene assay. In luciferase reporter gene assay, skatole and Guava coeur (registered trademark) were used by being mixed with a sample. The Fold Increase value in the test in which Guava coeur was not mixed was regarded as 1, and the ratio of the Fold Increase value in the test in which Guava coeur was mixed was obtained. The results are shown in Figure 6. The effect of concentration-dependently reducing the response of OR2L3 to skatole exerted by Guava coeur was shown.

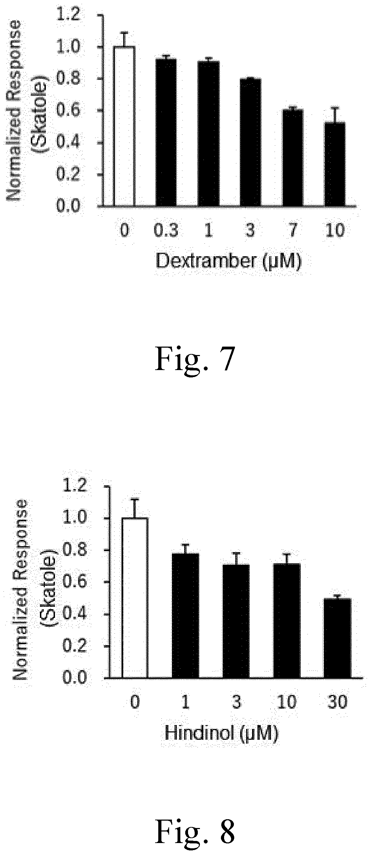

Guava coeur in the evaluation of the effect of suppressing the response of OR2L3 was replaced by Dextramber (registered trademark), and the test was conducted in the same manner. The results are shown in Figure 7. The effect of concentration-dependently reducing the response of OR2L3 to skatole exerted by Dextramber was shown. Similarly, Guava coeur was replaced by Hindinol (registered trademark) and the test was conducted. The results are shown in Figure 8. The effect of concentration-dependently reducing the response of OR2L3 to skatole exerted by Hindinol was shown.

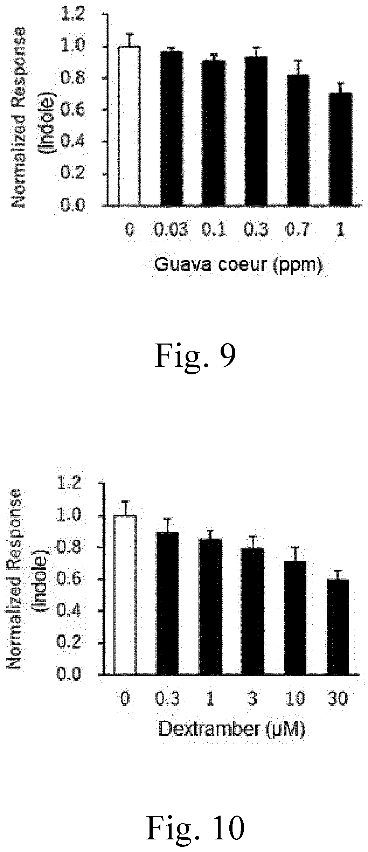

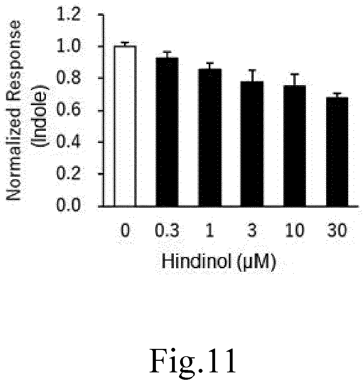

Regarding the unpleasant odor in the evaluation of the effect of suppressing the response of OR2L3, skatole was replaced by indole, and the test was conducted in the same manner. The results obtained by using Guava coeur, Dextramber and Hindinol, respectively, for Group A are shown in Figures 9 to 11. The effects of concentration-dependently reducing the response of OR2L3 to indole exerted by Guava coeur, Dextramber and Hindinol were shown.

In the evaluation of the effect of suppressing the response of OR2L3, the test was further conducted using combinations in which Guava coeur was replaced by another material of Group A and the skatole as the unpleasant odor was replaced by α-terpineol, 4-terpineol or p-methylacetophenone. The effects of concentration-dependently reducing the response of OR2L3 to the respective unpleasant odors exerted by all the materials of Group A were shown.

### [Example 3]

### Evaluation of unpleasant odor suppression ability of Group A

The unpleasant odor suppression ability of test substances having activity to inhibit receptor action was confirmed by sensory evaluation. A cotton ball on which 10 µL of skatole or indole diluted 100-fold with triethyl citrate as an unpleasant odor and 1 µL of a test substance were dropped was put into a plastic bottle (OZO-40 manufactured by Takemoto Yohki Co., Ltd.). The bottle was allowed to stand at room temperature for 1 hour to sufficiently volatilize odor molecules therein. The sensory evaluation test was conducted by a panel consisting of 20 persons, who made evaluation as follows: the odor strength in the case where the unpleasant odor was dropped solely was rated as 8, and the unpleasant odor strength in the case where the test substance was mixed was rated as from 0 (the unpleasant odor is not smelled) to 10 (the unpleasant odor is smelled very strongly). The average value was calculated from obtained numerical values. The results are shown in Table 1.

Humus Ether (registered trademark), which suppresses the response of OR2L3 to skatole, significantly suppressed the strength of skatole odor. This suppression of skatole was remarkable when compared to the cases of using a control substance (4-t-butylcyclohexanol, hexyl salicylate) which did not suppress the response of OR2L3 to skatole. Further, the case of indole was examined, and Humus Ether also suppressed the strength of indole odor. Note that when the effect of suppressing the unpleasant odors was tested also with respect to other substances that suppress the response of OR2L3 (Guava coeur, Pamplefix, Ambretone, Dextramber, Hindinol, Ringonol (each one is registered trademark), cardamom oil, clary sage oil, mandarin oil and spearmint oil), it became clear that all the substances suppress the unpleasant odors.

**Table 1**

| Fragrance substance | Skatole strength | Indole strength |
|---|---|---|
| Unpleasant odor alone | 8.0 | 8.0 |
| Humus Ether | 3.3 | 3.1 |
| Guava Coeur | 3.0 | 3.3 |
| Pamplefix | 2.5 | 2.4 |
| Ambretone | 4.2 | 3.6 |
| Dextramber | 4.0 | 3.9 |
| Hindinol | 3.6 | 4.1 |
| Ringonol | 3.9 | 3.7 |
| Cardamom oil | 4.5 | 4.0 |
| Clary sage oil | 3.3 | 3.2 |
| Mandarin oil | 3.6 | 4.2 |
| Spearmint oil | 3.2 | 3.0 |
| 4-t-butylcyclohexanol | 6.3 | 6.0 |
| Hexyl salicylate | 6.9 | 6.7 |

### [Example 4]

### <Evaluation of ability to suppress unpleasant odor using combination of Group A and Group B>

Fragrance compositions of Fragrance 1 to Fragrance 41 were prepared according to the formulations in Tables 2-6 below. DPG in the tables means dipropylene glycol. As shown in Table 7, Group B was divided into five groups, which were respectively referred to as Group B 1 to Group B 5 and added to the fragrance compositions. Note that the way of dividing the group and the ratio in the group are optional and not limited by Table 7. The floral-fruity base in Tables 2-6 was prepared according to Table 8.

Regarding these fragrance compositions, the ability to suppress the unpleasant odor was confirmed by means of sensory evaluation. As the unpleasant odor, a model fecal odor containing skatole, indole, butyric acid, p-cresol and dimethyl trisulfide (Lin et al., Environ. Sci. Technol., 2013, 47(14), pp. 7876-7882) was diluted 100-fold, and 10 µL thereof was dropped on a cotton ball, and as a test substance, 1 µL of one of Fragrance 1 to Fragrance 41 was dropped on the cotton ball. Then the cotton ball was put into a plastic bottle (OZO-40 manufactured by Takemoto Yohki Co., Ltd.). The bottle was allowed to stand at room temperature for 1 hour to sufficiently volatilize odor molecules therein. The sensory evaluation test was conducted by a panel consisting of 20 persons, who made evaluation as follows: the unpleasantness in the case where the unpleasant odor was dropped solely was rated as -3, and the unpleasantness in the case where the test substance was mixed was rated as from -4 (extremely unpleasant) to +4 (extremely pleasant). The average value was calculated from obtained numerical values. The results are shown in Tables 2-6.

The fragrance composition containing Humus Ether (registered trademark), which suppresses the response of OR2L3, suppressed the unpleasantness of the model fecal odor. In addition, it became clear that superior effects of suppressing the model fecal odor are exerted by combined use of Group B 1. This suppression of the model fecal odor was remarkable when compared to the cases of mixing a control substance (4-t-butylcyclohexanol, hexyl salicylate). Note that when the effect of suppressing the unpleasant odors was tested also with respect to other substances that suppress the response of OR2L3 (Guava coeur, Pamplefix, Ambretone, Dextramber, Hindinol, Ringonol (each one is registered trademark), cardamom oil, clary sage oil, mandarin oil and spearmint oil), it became clear that all the substances suppress the unpleasantness of each unpleasant odor and that more suppression effects are exerted by further using Group B 1 in combination.

Group B 1 in the test was replaced by Group B 2, and a test was similarly conducted. As a result, it became clear that superior effects of suppressing the unpleasantness of the model fecal odor are exerted by combined use of Group B 2 as in the case of using Group B 1.

Group B 1 was replaced by Group B 3, 4 or 5 and tests were similarly conducted. It became clear that superior effects of suppressing the unpleasantness of the model fecal odor are exerted by combined use of Group B 3, 4 or 5 as in the case of using Group B 1.

**Table 2**

| Fragrance component | Fragrance 1 | Fragrance 2 | Fragrance 3 | Fragrance 4 | Fragrance 5 | Fragrance 6 | Fragrance 7 | Fragrance 8 | Fragrance 9 |
|---|---|---|---|---|---|---|---|---|---|
| Humus Ether 10 mass% DPG solution | 0.1 | 0.1 | 0.1 | | | | | | |
| Guava coeur 0.1% DPG solution | | | | 0.1 | 0.1 | 0.1 | | | |
| Pamplefix 10 mass% DPG solution | | | | | | | 1.0 | 1.0 | 1.0 |
| Group B 1 | | | | | | | | | |
| Group B 2 | | 1.5 | 1.5 | | 1.5 | 1.5 | | 1.5 | 1.5 |
| Floral-fruity base | | | | | Balance | | | | |
| Total | 100 | 100 | 100 | 100 | 100 | 100 | 100 | 100 | 100 |
| Level of pleasantness/unpleasantness of unpleasant odor | 2.5 | 3.2 | 3.0 | 2.8 | 3.3 | 3.0 | 3.1 | 3.5 | 3.4 |

**Table 3**

| Fragrance component | Fragrance 10 | Fragrance 11 | Fragrance 12 | Fragrance 13 | Fragrance 14 | Fragrance 15 | Fragrance 16 | Fragrance 17 | Fragrance 18 |
|---|---|---|---|---|---|---|---|---|---|
| Ambretone | 1.0 | 1.0 | 1.0 | | | | | | |
| Dextramber 10 mass% DPG solution | | | | 0.5 | 0.5 | 0.5 | | | |
| Hindinol | | | | | | | 0.1 | 0.1 | 0.1 |
| Group B 1 | | 1.5 | 1.5 | | 1.5 | | | 1.5 | |
| Group B 2 | | | | | | 1.5 | | | 1.5 |
| Floral-fruity base | | | | | Balance | | | | |
| Total | 100 | 100 | 100 | 100 | 100 | 100 | 100 | 100 | 100 |
| Level of pleasantness/unpleasantness of unpleasant odor | 1.4 | 1.7 | 1.5 | 1.9 | 2.2 | 2.0 | 1.7 | 2.2 | 1.9 |

**Table 4**

| Fragrance component | Fragrance 19 | Fragrance 20 | Fragrance 21 | Fragrance 22 | Fragrance 23 | Fragrance 24 | Fragrance 25 | Fragrance 26 | Fragrance 27 |
|---|---|---|---|---|---|---|---|---|---|
| Ringonol 0.5 mass% DPG solution | 0.5 | 0.5 | 0.5 | | | | | | |
| Cardamom oil | | | | 0.5 | 0.5 | 0.5 | | | |
| Clary sage oil | | | | | | | 1.0 | 1.0 | 1.0 |
| Group B 1 | | 1.5 | | | 1.5 | | | 1.5 | |
| Group B 2 | | | 1.5 | | | 1.5 | | | 1.5 |
| Floral-fruity base | | | | | Balance | | | | |
| Total | 100 | 100 | 100 | 100 | 100 | 100 | 100 | 100 | 100 |
| Level of pleasantness/unpleasantness of unpleasant odor | 1.5 | 1.8 | 1.7 | 1.1 | 1.5 | 1.2 | 2.7 | 3.2 | 3.0 |

**Table 5**

| Fragrance component | Fragrance 28 | Fragrance 29 | Fragrance 30 | Fragrance 31 | Fragrance 32 | Fragrance 33 |
|---|---|---|---|---|---|---|
| Mandarin oil | 1.0 | 1.0 | 1.0 | | | |
| Spearmint oil | | | | 1.0 | 1.0 | 1.0 |
| Group B 1 | | 1.5 | | | 1.5 | |
| Group B 2 | | | 1.5 | | | 1.5 |
| Floral-fruity base | | | Bal | ance | | |
| Total | 100 | 100 | 100 | 100 | 100 | 100 |
| Level of pleasantness/unpleasantness of unpleasant odor | 1.5 | 2.0 | 1.9 | 3.3 | 3.6 | 3.5 |

**Table 6**

| Fragrance component | Fragrance 34 | Fragrance 35 | Fragrance 36 | Fragrance 37 | Fragrance 38 | Fragrance 39 | Fragrance 40 | Fragrance 41 |
|---|---|---|---|---|---|---|---|---|
| 4-t-butylcyclohexanol | 0.1 | 0.1 | 0.1 | | | | | |
| Hexyl salicylate | | | | 0.1 | 0.1 | 0.1 | | |
| Group B 1 | | 1.5 | | | 1.5 | | 1.5 | |
| Group B 2 | | | 1.5 | | | 1.5 | | 1.5 |
| Floral-fruity base | | | | | Balance | | | 100 |
| Total | 100 | 100 | 100 | 100 | 100 | 100 | 100 | |
| Level of pleasantness/unpleasantness of unpleasant odor | -2.0 | -1.3 | -1.5 | -1.3 | -1.8 | -1.5 | 1.0 | 1.0 |

**Table 7**

| Group | Group B Compound name |
|---|---|
| Group B 1 | Ethyl amyl ketone |
| | (2S,4R)-tetrahydro-4-methyl-2-(2-methylpropenyl)-2H-pyran |
| | 3-methyl-2-(cis-2-pentenyl)-2-cyclopenten-1-one |
| | 2-methoxynaphthalene |
| Group B 2 | Allyl hexanoate |
| | Ethyl hexanoate |
| | Ethyl isovalerate |
| | Isoamyl acetate |
| | Ethyl 2-methylvalerate |
| | Prenyl acetate |
| Group B 3 | Citronella oil |
| | Grapefruit oil |
| | Lime oil |
| Group B 4 | Chamomile oil |
| | Eucalyptus oil |
| | Peppermint oil |
| | Coriander oil |
| | Nutmeg oil |
| | p-mentha-1,8-diene-7-al |
| Group B 5 | Galbanum oil |
| | Cis-3-hexen-1-ol |
| | Cis-3-hexenyl acetate |
| | Methyl-2-nonynoate |
| | Cis-2-methyl-4-propyl-1,3-oxathiane |
| | 2-phenylpropanal |

**Table 8**

| <Floral-fruity base> | |
|---|---|
| Fragrance component | Parts by mass (%) |
| Cyclaprop | 4.5 |
| Galaxolide | 18.0 |
| Geraniol | 6.5 |
| Hedione | 14.0 |
| Hexyl acetate | 6.0 |
| Hexyl cinnamaldehyde | 6.0 |
| Linalool | 13.0 |
| Orbitone | 13.0 |
| Phenethyl alcohol | 6.0 |
| γ-undecalactone | 3.0 |
| Rose base (manufactured by Takasago International Corporation) | 10.0 |
| Total | 100.0 |

### [Example 5]

### <Gel air freshener>

A floral-fruity-note fragrance composition containing Humus Ether (registered trademark) (0.01%) and Group B 1 (1.5%) (Fragrance 2 in Table 2) was used as the fragrance composition, and a gel air freshener was produced by the ordinary method according to the formulation in Table 9 below. Note that a fragrance composition obtained according to a formulation in which Humus Ether was replaced by a compound of Group A and Group B was replaced by a compound of Group B was also used to similarly produce a gel air freshener according to the ordinary method.

**Table 9**

| Component | Parts by mass (%) |
|---|---|
| Carrageenan | 2.0 |
| Locust bean gum | 0.2 |
| Propylene glycol | 2.0 |
| POE (20) sorbitan monostearate | 0.5 |
| Fragrance composition | 5.0 |
| Purified water | Remainder |
| Total | 100.0 |

### [Example 6]

### <Body soap>

A citrus-note fragrance composition containing Guava coeur (registered trademark) (0.0001%), Group B 3 (1.5%) and Citrus base (remainder) was used as the fragrance composition, and a body soap was produced by the ordinary method. The formulation of Citrus base is described in Table 10, and the formulation of the body soap is described in Table 11. Note that a fragrance composition obtained according to a formulation in which Guava coeur was replaced by a compound of Group A and Group B was replaced by a compound of Group B was also used to similarly produce a body soap according to the ordinary method.

**Table 10**

| <Citrus base> | |
|---|---|
| Fragrance component | Parts by mass (%) |
| Citral | 2.5 |
| Galaxolide | 6.0 |
| Geraniol | 5.0 |
| Hedione | 22.0 |
| Heliobouquet | 2.5 |
| Hexyl cinnamaldehyde | 13.0 |
| Lemon oil | 6.0 |
| Linalool | 10.0 |
| Linalyl acetate | 5.0 |
| Orange oil | 18.0 |
| Lemon base (manufactured by Takasago International Corporation) | 10.0 |
| Total | 100.0 |

**Table 11**

| <Body soap> | |
|---|---|
| Component | Parts by mass (%) |
| Triethanolamine | 9.00 |
| Lauric acid | 6.00 |
| Myristic acid | 9.00 |
| Disodium laureth sulfosuccinate (1E.O.) (42%) | 10.00 |
| (C8-16) Alkyl glucoside | 8.00 |
| Glyceryl laurate | 1.00 |
| 2-hydroxyethyl distearate | 2.50 |
| Coconut oil fatty acid diethanolamide | 3.00 |
| Propylene glycol | 5.00 |
| Dibutylhydroxytoluene | 0.05 |
| Disodium edetate | 0.10 |
| Ethyl paraoxybenzoate | 0.20 |
| Methyl paraoxybenzoate | 0.10 |
| Fragrance composition | 0.95 |
| Purified water | Remainder |
| Total | 100.00 |

### [Example 7]

### <Liquid softener>

A floral-balsamic-note fragrance composition containing Pamplefix (registered trademark) (0.005%), Group B 4 (5%) and Floral-balsamic base (remainder) was used as the fragrance composition, and a liquid softener was produced by the ordinary method. The formulation of Floral-balsamic base is described in Table 12, and the formulation of the liquid softener is described in Table 13. Note that a fragrance composition obtained according to a formulation in which Pamplefix was replaced by a compound of Group A and Group B was replaced by a compound of Group B was also used to similarly produce a liquid softener according to the ordinary method.

**Table 12**

| <Floral-balsamic base> | |
|---|---|
| Fragrance component | Parts by mass (%) |
| Acetanisole | 1.0 |
| Benzyl salicylate | 7.0 |
| Calone | 0.5 |
| Cyclamen aldehyde | 3.0 |
| Floralozone | 1.5 |
| Galaxolide | 17.0 |
| Geraniol | 4.0 |
| Hedione | 30.0 |
| Isoeugenol | 1.5 |
| γ-nonalactone | 0.5 |
| Orbitone | 21.0 |
| Phenethyl alcohol | 3.0 |
| Jasmine base (manufactured by Takasago International Corporation) | 10.0 |
| Total | 100.0 |

**Table 13**

| <Liquid softener> | |
|---|---|
| Component | Parts by mass (%) |
| Imidazoline-type cationic surfactant | 6.00 |
| Ethylene glycol | 2.00 |
| Polyoxyethylene oleyl cetyl ether | 0.20 |
| Fragrance composition | 1.00 |
| Purified water | Remainder |
| Total | 100.00 |

### INDUSTRIAL APPLICABILITY

It is expected that the use of the fragrance composition according to the present invention can contribute to the reduction in unpleasantness of at least one of fecal odor, body odor, tobacco odor, breath odor and musty odor.

## Claims

1. Use of a fragrance composition for suppressing a response strength of at least one olfactory receptor polypeptide, which is selected from the group consisting of OR2L3 and polypeptides that comprise an amino acid sequence having at least 80% identity to an amino acid sequence of OR2L3 and are responsive to an unpleasant odor-causing substance, to the unpleasant odor-causing substance,
wherein the fragrance composition comprises a fragrance component comprising at least one selected from the group consisting of materials of Group A below:
Group A: methyl fenchol, ethyl 3-mercapto-2-methylbutyrate, dimethyl-2-(1-phenylethyl)cyclopropylmethanol, 5-cyclohexadecenone, 1-(2,2,6-trimethylcyclohexyl)hexan-3-ol, 2-methyl-4-(2,2,3-trimethyl-3-cyclopentenyl)-2-buten-1-ol, p-mentha-8-thiol-3-one, cardamom oil, clary sage oil, mandarin oil and spearmint oil.

2. The use according to claim 1, wherein the unpleasant odor-causing substance is at least one selected from the group consisting of skatole, indole, α-terpineol, 4-terpineol and p-methylacetophenone.

3. The use according to claim 1 or 2, wherein the unpleasant odor is at least one selected from the group consisting of fecal odor, body odor, tobacco odor, breath odor and musty odor.

4. The use according to any one of claims 1 to 3, wherein the fragrance component further comprises at least one selected from the group consisting of materials of Group B below:
Group B: methyl-2-nonynoate, p-mentha-1,8-diene-7-al, 2-phenylpropanal, ethyl amyl ketone, cis-2-methyl-4-propyl-1,3-oxathiane, ethyl hexanoate, ethyl 2-methylvalerate, tetrahydro-4-methyl-2-(2-methylpropenyl)-2H-pyran, (2S,4R)-tetrahydro-4-methyl-2-(2-methylpropenyl)-2H-pyran, allyl hexanoate, isoamyl acetate, 3-methyl-2-(cis-2-pentenyl)-2-cyclopenten-1-one, cis-3-hexenyl acetate, prenyl acetate, 2-methoxynaphthalene, cis-3-hexen-1-ol, ethyl isovalerate, grapefruit oil, chamomile oil, lime oil, galbanum oil, citronella oil, peppermint oil, eucalyptus oil, nutmeg oil and coriander oil.

5. The use according to claim 4, wherein the fragrance composition comprises 0.000001 to 50% by mass of the fragrance component of Group A and 0.0001 to 50% by mass of the fragrance component of Group B.

## Patentansprüche

1. Verwendung einer Duftzusammensetzung zur Unterdrückung der Reaktionsstärke mindestens eines Geruchsrezeptor-Polypeptids, das ausgewählt ist aus der Gruppe bestehend aus OR2L3 und Polypeptiden, die eine Aminosäuresequenz umfassen, die zu mindestens 80% mit einer Aminosäuresequenz von OR2L3 identisch ist und die auf eine unangenehm riechende Substanz reagieren, auf die unangenehm riechende Substanz,
wobei die Duftzusammensetzung eine Duftkomponente umfasst, die mindestens eines umfasst, ausgewählt aus der Gruppe bestehend aus den folgenden Stoffen der Gruppe A:
Gruppe A: Methylfenchol, Ethyl-3-mercapto-2-methylbutyrat, Dimethyl-2-(1-phenylethyl)cyclopropylmethanol, 5-Cyclohexadecenon, 1-(2,2,6-Trimethylcyclohexyl)hexan-3-ol, 2-Methyl-4-(2,2,3-trimethyl-3-cyclopentenyl)-2-buten-1-ol, p-Mentha-8-thiol-3-on, Kardamomöl, Muskatellersalbeiöl, Mandarinenöl und Grüne-Minze-Öl.

2. Verwendung gemäß Anspruch 1, wobei die unangenehm riechende Substanz mindestens eine ist, ausgewählt aus der Gruppe bestehend aus Skatol, Indol, α-Terpineol, 4-Terpineol und p-Methylacetophenon.

3. Verwendung gemäß Anspruch 1 oder 2, wobei der unangenehme Geruch mindestens einer ist, ausgewählt aus der Gruppe bestehend aus Fäkalgeruch, Körpergeruch, Tabakgeruch, Mundgeruch und muffigem Geruch.

4. Verwendung gemäß mindestens einem der Ansprüche 1 bis 3, wobei die Duftkomponente ferner mindestens eine umfasst, ausgewählt aus der Gruppe bestehend aus den folgenden Stoffen der Gruppe B:
Gruppe B: Methyl-2-nonynoat, p-Mentha-1,8-dien-7-al, 2-Phenylpropanal, Ethylamylketon, cis-2-Methyl-4-propyl-1,3-oxathian, Ethylhexanoat, Ethyl-2-methylvalerat, Tetrahydro-4-methyl-2-(2-methylpropenyl)-2H-pyran, (2S,4R)-Tetrahydro-4-methyl-2-(2-methylpropenyl)-2H-pyran, Allylhexanoat, Isoamylacetat, 3-Methyl-2-(cis-2-pentenyl)-2-cyclopenten-1-on, cis-3-Hexenylacetat, Prenylacetat, 2-Methoxynaphthalin, cis-3-Hexen-1-ol, Ethylisovalerat, Grapefruitöl, Kamillenöl, Limettenöl, Galbanumöl, Citronellaöl, Pfefferminzöl, Eukalyptusöl, Muskatnussöl und Korianderöl.

5. Verwendung gemäß Anspruch 4, wobei die Duftzusammensetzung 0,000001 bis 50 Massenprozent der Duftkomponente der Gruppe A und 0,0001 bis 50 Massenprozent der Duftkomponente der Gruppe B umfasst.

## Revendications

1. Utilisation d'une composition parfumante pour supprimer une intensité de réponse d'au moins un polypeptide de récepteur olfactif, qui est sélectionné dans le groupe constitué d'OR2L3 et de polypeptides qui comprennent une séquence d'acides aminés présentant au moins 80 % d'identité avec une séquence d'acides aminés d'OR2L3 et présentant une sensibilité à une substance générant une odeur désagréable, à la substance générant une odeur désagréable,
dans laquelle la composition parfumante comprend un ingrédient parfumant comprenant au moins une matière sélectionnée dans le groupe constitué des matières du groupe A ci-dessous :
groupe A : méthyl fenchol, éthyl 3-mercapto-2-methylbutyrate, diméthyl-2-(1-phenyléthyl)cyclopropylméthanol, 5-cyclohexadecenone, 1-(2,2,6-triméthylcyclohexyl)hexan-3-ol, 2-méthyl-4-(2,2,3-triméthyl-3-cyclopentenyl)-2-buten-1-d. p-mentha-8-thiol-3-one, huile de cardamome, huile de sauge sclarée, huile de mandarine et huile de menthe verte.

2. Utilisation selon la revendication 1, dans laquelle la substance générant une odeur désagréable est au moins une substance sélectionnée dans le groupe constitué de skatole, d'indole, d'α-terpinéol, de 4-terpinéol et de p-méthylacétophénone.

3. Utilisation selon la revendication 1 ou la revendication 2, dans laquelle l'odeur désagréable est au moins une odeur sélectionnée dans le groupe constitué de l'odeur fécale, de l'odeur corporelle, de l'odeur de tabac, de l'odeur de l'haleine et de l'odeur de moisi.

4. Utilisation selon l'une quelconque des revendications 1 à 3, dans laquelle l'ingrédient parfumant comprend en outre au moins une matière sélectionnée dans le groupe constitué des matières du groupe B ci-dessous :
groupe B : méthyl-2-nonynoate, p-mentha-1,8-diene-7-al, 2-phenylpropanal, éthyl amyl ketone, cis-2-méthyl-4-propyl-1,3-oxathiane, éthyl hexanoate, éthyl 2-methylvalerate, tétrahydro-4-méthyl-2-(2-méthylpropenyl)-2H-pyran, (2S,4R)-tétrahydro-4-méthyl-2-(2-méthylpropenyl)-2H-pyran, allyl hexanoate, isoamyl acétate, 3-méthyl-2-(cis-2-pentenyl)-2-cyclopenten-1-one, cis-3-hexenyl acetate, prenyl acétate, 2-méthoxynaphthalène, cis-3-hexen-1-ol, éthyl isovalerate, huile de pamplemousse, huile de camomille, huile de citron vert, huile de galbanum, huile de citronnelle, huile de menthe poivrée, huile d'eucalyptus, huile de noix de muscade et huile de coriandre.

5. Utilisation selon la revendication 4, dans laquelle la composition parfumante comprend 0,000001 à 50 % en masse de l'ingrédient parfumant du groupe A et 0,0001 à 50 % en masse de l'ingrédient parfumant du groupe B.
